# EUROPEAN PATENT APPLICATION

(11) **EP 2 077 126 A1**
(43) Date of publication of application: **08.07.2009**
(21) Application number: 08290010.1
(22) Date of filing: 07.01.2008
(51) Int. Cl.: A61L 27/54, A61L 27/12, A61L 24/00, A61L 24/02, A61K 9/00, A61K 31/00

(54) **Analgesic apatitic calcium-phosphate cement**

(71) Applicant: Graftys, 13854 Aix en Provence Cedex 3 (FR)
(72) Inventor: Leguen, Hervé, 44000 Nantes (FR); Cavagna, Rémi, 56270 Ploemeur (FR); Khairoun, Ibrahim, 44200 Nantes (FR); Verron, Elise, 44120 Vertou (FR); Janvier, Pascal, 44000 Nantes (FR); Gauthier, Olivier, 44220 Suce Sur Erder (FR); Bouler, Jean-Michel, 44470 Carquefou (FR)
(74) Representative: Kling, Simone

(57) **Abstract**

The present invention concerns a composition useful as bone substitute comprising one or more calcium-phosphate compounds in association with an analgesic.

It also refers to a preparation process of said composition, a preparation process of a drug-combined device comprising said composition, the drug combined device thus obtained, a kit comprising said composition and the use of said composition for the preparation of a drug-combined device useful for filling a bony defect caused in the iliac crest by collection of auto-graft bone, as a scaffold for tissue engineering and to produce a dental or bony implant.

## Description

### [Technical Field]

The invention relates to a bioresorbable calcium-phosphate composition having analgesic properties, in particular useful as a bone substitute, capable of easing the pain associated with orthopaedic operations notably those associated with the collection of auto-graft bone.

### [Background of the invention]

The innervations of bone are rich and complex. Therefore, orthopaedic operations are often associated with strong pain.

The pain following heavy orthopaedic operations is one of the most intense observed in postoperative period. Present during rest, the pain rises markedly upon movement. The pain is moderate to severe during the 48 to 72 hours following the operation, and subsides rapidly afterwards. It thus constitutes an important barrier for early re-education.

Postoperative bone pain is a model for strong pain by excessive nociception (peripheral bone and articular receptors). The operative intervention is thus at the origin of a complex inflammatory process, in fact an inflammatory soup which will contribute to a steady stimulation of the peripheral receptors.

To date, different approaches to treat or prevent such pain have been developed.

Analgesics may be administered by general route (intravenous or per os). In this approach, conventional analgesics (paracetamol, tramadol, codeine, nefopam...), Nonsteroidal Antiinflammatory Drugs (NSAIDs), AINS (Ketoprofen, Indomethacin) or morphine derived products are generally used.

However, the administration by general route often implies large dosages and is thus likely to entrain side effects. Some patients further may not be eligible for such a treatment because they present a contraindication to the products used such as gastric ulcer or chronic respiratory insufficiency.

As an alternative, techniques based on the local administration of local anaesthetics *en bolus* or via catheter have also been developed.

Indeed, the continuous and controlled administration of local anaesthetics very close to the chirurgical site is an approach which could allow for a reduction of the nociceptive afferences by blocking them in the periphery.

In dental surgery, several studies have analysed the administration to the bone of local anaesthetics for dental care (6), with interesting results and innovative administration modalities (7).

In 2002, a team of the University Hospital of Bordeaux, France, compared the effectiveness of an intrabone injection of lidocaine versus an antalgic protocol comprising nalbuphine and paracetamol during percutaneous vertebroplastics. They showed a comparable effectiveness with serum levels of lidocaine much below the toxic level (8).

The integration technique avoids loss of properties of neither the calcium phosphate cements (CPC) nor the local anesthetics (lidocaine or bupivacaine).

It has been attempted (9) (10) to introduce local anaesthetics directly intra-articularily. A catheter under constant flow was placed surgically within the articulation at the end of total-knee arthroplasty. In this study, the efficiency was poor, probably also related to the bleeding and the presence of drains.

The separate local administration of analgesics (intra-articular catheter) however entails an additional risk of infection (septic arthritis).

The aim of the invention was to provide a means to ease pain associated with orthopaedic or dental surgery with limited side effects and risk of infection.

### [Summary of the invention]

According to the invention is proposed a calcium-phosphate based bioresorbable composition, usable as a bone or a dental substitute, which comprises an analgesic in a form suitable to be released *in situ*.

The first object of the invention thus relates to a composition useful as bone substitute comprising one or more calcium-phosphate compounds in association with an analgesic.

The second object of the invention relates to a preparation process of a composition according to the invention, comprising the following steps:
(a) providing a calcium-phosphate powder, a liquid and an analgesic;
(b) mixing of the components to obtain a suspension; and
(c) removing the liquid from the suspension to obtain a solid; and
(d) optionally compressing and grinding the solid obtained into a calcium-phosphate powder charged with the analgesic.

The third object of the invention relates to a composition obtainable according to the process of the invention.

The fourth object of the invention relates to a preparation process of a drug combined device comprising the following steps:
(i) mixing of a composition according to the invention with an appropriate amount of an aqueous medium ;
(ii) forming the mixture into a suitable form ; and,
(iii) setting of the mixture into a solid drug-combined device.

The fifth object of the invention relates to a drug-combined device comprising a composition according to the invention.

The sixth object of the invention relates to a kit comprising a composition according to the invention, an aqueous medium and optionally further one or more calcium phosphate compounds.

The seventh object of the invention relates to the use of a composition according to the invention for the preparation of a drug-combined device useful for filling a bony defect caused in the iliac crest by collection of auto-graft bone.

The eighth object of the invention is the use of the composition according to the invention as an analgesic bone cement.

The ninth object of the invention relates to the use *in vitro* or *ex vivo* of a composition according to the invention, as a scaffold for tissue engineering.

The tenth object of the invention relates to the use *in vitro* or *ex vivo* of a composition according to the invention to produce a dental or bony implant.

The eleventh object of the invention is a method of treatment comprising the injection in a dental or bony defect of an injectable composition according to the invention.

The described composition allows for the administration of an analgesic in situ, in particular relieving pain following orthopaedic and dental surgery.

The composition allows for the administration of low dosages of analgesics, reducing thus the risk of side effects. The analgesic is released in situ from the composition in a controlled way, over a period commensurate with the period of postoperative pain.

It is compatible with various analgesics and can be envisaged for the treatment of a large panel of patients.

Therefore, the composition provides further for drug combined devices such as bone substitute which allow a postoperational pain treatment without any further separate intervention, thus reducing the risk of infection and enhancing the patient's comfort.

### [Definitions]

As used herein, "bioresorbable" means whose degradative products are metabolized *in vivo* or excreted from the body via natural pathways.

A "bioceramic" is a biocompatible and preferably bone growth stimulating ceramic material which may be used for reconstructive bone surgery and dental implants.

A "cement" is a dough resulting from the mixing of a pulverulent solid phase and an aqueous medium and the hardened material obtained after setting.

The "setting" of a cement means the hand-off auto-hardening at room or body temperature of the paste resulting from the mixing of the solid phase and the aqueous medium.

An "injectable cement" or a "cement in a form suitable to be injected" means a cement paste sufficiently fluid to flow through a needle with a diameter of a few millimetres, preferably between 1 and 5 mm.

A "calcium-phosphate compound" is a compound containing calcium ions and ortho-phosphate (PO₄³⁻), metaphosphate or pyrophosphate (P₂O₇⁴⁻) groups, optionally water and occasionally small amounts of other ions, such as hydrogen and hydroxyde. Such calcium phosphate compounds include hydroxyapatite (HA) Ca₁₀(PO₄)₆(OH)₂ ; amorphous calcium phosphate (ACP), Caₓ(PO₄)_{y}.H₂O ; monocalcium phosphate monohydrate (MCPH), CaH₄(PO₄)₂.H₂O ; dicalcium phosphate dihydrate (DCPD), CaHPO₄.2H₂O, also called brushite ; dicalcium phosphate anhydrous (DCPA), CaHPO₄ ; precipitated or calcium-deficient apatite (CDA), (Ca,Na)₁₀(PO₄,HPO₄)₆(OH)₂ ; α- or β- tricalcium phosphate (α-TCP, β-TCP), Ca₃(PO₄)₂ ; and tetracalcium phosphate (TTCP), Ca₄P₂O₉.

An "apatitic" calcium phosphate crystallises in the hexagonal system and has the formula Ca₁₀₋ₓ(PO₄)₆₋ₓ,(OH, Cl, F, (CO₃)_{1/2})₂₋ₓ with x ≥ 1.

A solid is said "amorphous" when it is without crystalline structure.

The "compressive strength" is the maximal compressive stress supported by a sample upon failure and is expressed in MPa.

A "microparticle" has a diameter less than 1 mm, preferably between 100nm and 300 µm, preferably 1 and 250 µm, more preferably between 40 and 80 µm.

An "implant" is medical device introduced in the body to replace in part or entirely a biological structure such as a tooth, a joint, a bone or a cartilage.

A "minimally invasive surgery" means a technique of surgery that does not require a large incision but a few centimetres incision, preferably ≤ 5 cm.

Dendrimers are high size arborescent (dendritic) polymers produced by iterative processes from molecules with at least three reactive sites.

Polysaccharides are a class of carbohydrates, such as starch and cellulose, consisting of a number of monosaccharides linked by glycosidic bonds.

### [Detailed Description of the invention]

### [The composition]

According to the most general definition, the invention is directed to a composition which comprises at least one calcium phosphate compound in association with an analgesic.

The concept and potential advantages of calcium phosphate cement (CPC) as a possible restorative material was first introduced by LeGeros et al in 1982 ("Apatitic Calcium Phosphates : Possible Restorative Materials", J Dent Res. 61(Spec Iss):343).

CPC have the following advantages: malleability allowing them to adapt to the defect's site and shape. The introduction of injectable calcium phosphate cements greatly improved the handling and delivery of the cements and opened up areas of new applications for the CPC.

CPC systems consist of a powder and an aqueous medium as a liquid component. The powder component is usually made up of one or more calcium phosphate compounds with or without additional calcium salts. Other additives are included in small amounts to adjust setting times, increase injectability, reduce cohesion or swelling time, and/or introduce macroporosity.

The liquid component may comprise or consist of one or more of the following: saline, deionized water, dilute phosphoric acid, dilute organic acids (acetic, citric, succinic acid), sodium phosphate (alkaline or neutral), sodium carbonate or bicarbonate, sodium alginate, sodium bicarbonate, sodium citrate, and/or sodium chondroitin sulphate.

The first object according to the invention concerns a composition useful as bone cement comprising or consisting of one or more calcium phosphate compounds in association with an analgesic.

The composition according to the invention may be in the form of a powder, preferably with a mean diameter of about between 0,2 µm and 100 µm; it may also be in form of granules, with a mean diameter preferably of about between 1 mm and 5 mm.

Upon use, the composition will generally be mixed with a liquid to form a dough, which may be put into a suitable form before it subsequently sets into a solid, as set out above.

ACP is the most soluble in the group of calcium phosphate compounds used in many CPCs. ACP can be made more or less stable (i.e. more or less soluble or more or less susceptible to transform to other calcium phosphates) depending on the ions incorporated in it. (LeGeros et al., (1973), "Amorphous calcium phosphates:synthetic and biological).

Preferably, the calcium phosphate compounds for the composition according to the invention are selected from the group consisting of ACP, MCPH, DCPD, DCPA, CDA, TTCP α-TCP and mixtures thereof.

In particular, the composition according to the invention comprises at least one above defined calcium phosphate compound selected from the group consisting of CDA, DCPD, DCPA, α-TCP or a mixture thereof.

In a preferred embodiment, the calcium-phosphate compounds of the composition according to the invention have a specific BET area, measured according to the Brunnauer Emmet Teller method (11), of between about 500 m².kg⁻¹ and 300 000 m².kg⁻¹, preferably between about 1000 m².kg⁻¹ and 100 000m².kg⁻¹ more preferably between about 5 000 m².kg⁻¹ and 50 000m².kg⁻¹.

In a preferred embodiment, the composition according to the invention comprises at least about 40%, preferably about 50%, more preferably about 60%, still more preferably about 70%, the most preferably about 80% by weight of α-TCP.

The composition according to the invention further contains an analgesic, in particular a morphine related substance.

Preferably the analgesic is a local anaesthetic. Local anaesthetics (LA) stop nervous transmission by blocking the sodium canal at the level of the anonal membranes (1).

Local anesthetic drugs act mainly by inhibiting sodium influx through sodium-specific ion channels in the neuronal cell membrane, in particular the so-called voltage-gated sodium channels. When the influx of sodium is interrupted, an action potential cannot arise and signal conduction is inhibited. The receptor site is thought to be located at the cytoplasmic (inner) portion of the sodium channel. Local anesthetic drugs bind more readily to "open" sodium channels, thus onset of neuronal blockade is faster in neurons that are rapidly firing. This is referred to as state dependent blockade.

Local anesthetics are weak bases (pKa between 7,6 et 8,9) and are usually formulated as the hydrochloride salt to render them water-soluble. At physiologic pH the protonated and unprotonated forms of the molecule exist in an equilibrium but only the unprotonated molecule diffuses readily across cell membranes. Once inside the cell, the local anesthetic will be in equilibrium, with the formation of the protonated, which does not readily pass back out of the cell. This is referred to as "ion-trapping". In the protonated form, the molecule binds to the local anaesthetic binding site on the inside of the ion channel near the cytoplasmic end.

Clinical local anesthetics belong to one of two classes: aminoamides and aminoester.

Synthetic local anesthetics are structurally related to cocaine. They differ from cocaine mainly in that they have no abuse potential and do not act on the sympathoadrenergic system, i.e. they do not produce hypertension or local vasoconstriction, with the exception of ropivacaine and mepivacaine that do produce weak vasoconstriction.

Local anesthetics in clinical use include amino esters such as benzocaine, chloroprocaine, cocaine, procaine and tetracaine, amino amides such as bupivacaine, levobupivacaine, lidocaine, mepivacaine, prilocaine, ropivacaine, articaine and trimecaine.

Due to an asymetric carbon atom, some of these molecules present levogyre and dextrogyre forms : bupivacaine and ropivacaine. The levogyre form is generally the less toxic isomer.

Other effects of local anesthetics are less well-known:
Local anesthetics inhibit the fixation of the substance P on its receptor at the level of the bone marrow. (2)
Local anesthetics present direct anti-inflammatory properties on the leuco-cyties fonctions. They possess antalgic properties by intravenous administration albeit the toxicity risk; continuously administrated intraveineously lidocaine allows for a reduction of the postoperative morphine intake and an early post operative rehabilitation (3).

Lidocaine is often used as an antiarrhythmic drug and has been studied extensively, but the effects of other local anesthetics are probably similar to those of lidocaine.

The analgesic may be simply mixed, adsorbed onto the surface of the mineral component or absorbed within their porous structure. Preferably, the analgesic is at least partially absorbed, since this spurs a controlled release of the analgesic from the composition over a long period.

The proportion of analgesic contained in composition according to the invention may vary largely depending on the application.

Generally speaking, the composition according to the invention will contain from 0,5 to 20 %, preferably 1 to 10% by weight of analgesic.

The composition may further include other components, such as bioceramics and polymers.

In a preferred embodiment, the composition according to the invention further comprises bioceramics. Preferably, said bioceramics are one or more sintered calcium phosphate compounds selected from the group consisting of hydroxyapatite (HA), alpha- and beta-tricalcium phosphate (α-TCP, β-TCP) and biphasic calcium phosphate (BCP) or a mixture thereof.

The most common method used to prepare calcium phosphate bioceramics, involves the use of powders prepared from aqueous solutions of the starting chemicals. These powders are compacted under high pressure (between 50MPa and 500 MPa) and then sintered at between 1000°C and 1300°C (See Jarcho, 1986). Biphasic calcium phosphate (BCP) is obtained when calcium-deficient biologic or synthetic apatites are sintered at or above 700°C. An apatite is considered calcium deficient when the Ca/P ratio is less than the stoichiometric value of 1.67 for pure calcium hydroxyapatite. Precipitates of hydroxyapatites can be made from an aqueous solution of Ca(NO₃)₂ and NaH₂PO₄. One method uses precipitates that are filtered and dried to form a fine particle powder. After calcination for 3 hours at 900°C, the powder is pressed into a final form and sintered at about 1050°C to 1200°C for 3 hours.

Bioceramics according to the invention are preferably in the form of granules or agglomerated granules. If they are intended for a 3D implant, the bioceramics may preferably be present in the form of cones, cylinders and sticks.

The composition according to the invention can further include one or more biocompatible and bioresorbable polymers. The inorganic component of the composition according to the invention allows an intimate bond with the native bone and osteogenic properties. The organic component allows macroporosity interconnected in the mineral matrix and improves the cohesion, the elasticity, the rheological properties and the injectability of the cement.

Biocompatible and bioresorbable polymers useful in the invention include, for example, a polymer from the linear polyester family, such as polylactic acid, polyglycolic acid or poly(ε)caprolactone and their associated copolymers, e.g. poly (lactide-co-glycolide) at all lactide to glycolide ratios, and both L-lactide or D,L-lactide; polyphosphazenes, dendrimers and polysaccharides; polyorthoester, polyanhydride, polydioxanone, hyaluronic acid and polyhydroxybutyrate and their salts and mixtures thereof.

Polyphosphazenes, dendrimers, polysaccharides, poly(ε)caprolactone and their salts and mixtures thereof are preferred as the organic component of the cement. In addition to their physical properties and good compressive strengths, these can be produced with appropriate resorption speed, hydrophilic properties and solubility. Then, this allows the control of their resorbability and the guided resorption-substitution of the composition according to the invention.

Polyphosphazenes are preferably selected from the group consisting of poly(ethyl oxybenzoate)phosphazene (PN-EOB), poly(propyl oxybenzoate) phosphazene (PN-POB), poly[bis(sodium carboxylatophenoxy)phosphazene] (Na-PCPP), poly[bis(potassium carboxylatophenoxy) phosphazene] (K-PCPP), poly[bis(ethyl alanato)phosphazene] (PA+IaP), poly[bis(carboxylatophenoxy) phosphazene] (acid-PCPP), and their salts and mixtures thereof.

Polysaccharides and their salts and mixtures thereof are more preferred polymers used in the organic component of the cement. Cellulose ethers and their salts and mixtures thereof are preferred polysaccharides used in the organic component of the cement, more preferably selected from the group consisting of hydroxypropylmethylcellulose (HPMC), carboxymethylcellulose (CMC)

Biocompatible and bioresorbable polymers can be used as fine powders, fibers or microparticles. Polymer microparticles can be microspheres or microcapsules, preferably encapsulating one or several excipients such as saccharose, glucose, water, a gas as air, or one or several pharmaceutically active substances as an antibiotic, an anti-inflammatory drug, an anti-cancer drug, a drug against osteoporosis, a growth factor or a mixture thereof. Encapsulating methods are well known by the one skilled in the art.

The organic component varies between 0.1 to 30% by weight of the total amount of the composition according to the invention.

Preferably, the ether cellulose amount varies from between 0,1 to 5, preferably 1 to 3%, more preferably 1 to 2% by weight of the total amount of the composition according to the invention.

The most preferred cement comprises an organic component consisting in HPMC or CMC or poly(ε)caprolactone or a mixture thereof.

### [Preparation process]

Another object of the present invention relates to a preparation process for charging the calcium phosphate compound with the analgesic, thus providing the composition according to the invention described above.

Preferably, the preparation process according to the invention comprises the following steps:
(a) providing a calcium-phosphate powder, a liquid and an analgesic ;
(b) mixing the components to obtain a suspension ;
(c) removing the liquid from the suspension to obtain a solid ; and
(d) optionally compressing, preferably in an isostatic manner, and grinding the solid obtained into a calcium-phosphate powder charged with the analgesic.

This process allows for the association, preferably at least partially by absorption, of the analgesic within the porous structure of the calcium phosphate which may lead to a controlled release of the drug from the obtained material.

In a preferred embodiment, step (c) is conducted by lyophilisation.

In a preferred embodiment, said calcium-phosphate powder in step (a) has a specific BET area between about 500 m².kg⁻¹ and 300 000 m².kg⁻¹, preferably between about 1 000 m².kg⁻¹ and 100 000 m².kg⁻¹, more preferably between about 5 000 m².kg⁻¹ and 50 000 m².kg⁻¹.

In a preferred embodiment, said calcium-phosphate powder in step (a) has a mean particle size comprised between about 0,2 µm and about 100 µm, preferably 10 µm and 90 µm, more preferably 20 µm and 80 µm.

In a preferred embodiment, said suspension in step ( b) comprises from about 0,5 to about 20 % by weight of analgesic, preferably about 1 to about 15 % by weight of analgesic, more preferably about 2 to about 10 % by weight of analgesic.

Preferably, the compression in step (d) is carried out at a pressure of between about 50 MPa to about 500 MPa, more preferably between about 100 MPa to about 200 MPa.

In a preferred embodiment, the calcium-phosphate powder charged with the analgesic obtained in step (d) has a particle size between about 1 µm and about 500 µm, preferably 10 µm and 400 µm, more preferably 100 µm and 200 µm.

There are several other options as to how and when incorporate the analgesic into the composition.

In particular, the drug may be incorporated immediately prior to use, during the manufacture of the cement dough, by adding the analgesic to the solid component or the liquid component prior to mixing.

This embodiment may be preferred in cases where the drug stability could be affected by dissolution. In this embodiment, the drug is preferably added to the composition according to the invention in the form of a powder.

According to another embodiment, the drug is introduced directly upon the preparation of the cement into the cement dough. This embodiment may be preferred in cases where the drug activity could be affected by interaction with the organic component, since it reduces the contact time.

According to a third embodiment, the drug, the solid component and the liquid component are mixed together simultaneously.

A further object of the present invention relates to a composition obtainable by the preparation process described above.

### [Preparation process of drug combined devices]

A further object of the present invention relates to the use of the composition according to the invention for the manufacture of drug combined devices.

More specifically, the preparation process of a drug combined device according to the invention comprises the following steps of:
(i) the mixing of a composition according to the invention in form of a powder with an appropriate amount of a aqueous medium ;
(ii) putting the mixture into a suitable form ; and
(iii) setting of the mixture into a solid drug-combined device.

Preferably, the mixture in step (i) is in a form suitable to be injected. In such a case, the aqueous medium may be a liquid or a gel. Injectable composition is useful to be injected in small and closed bone cavities, where it sets *in situ*.

The composition according to the invention is particularly useful as a calcium phosphate cement (CPC) associated with an analgesic.

Upon use, the composition according to the invention is mixed with an appropriate amount of an aqueous medium and hardens by hydraulic setting.

Preferably, the freshly prepared mixture is in a form suitable to be injected.

An appropriate aqueous medium includes one or more of the following: saline, deionized water, dilute phosphoric acid, dilute organic acids (acetic, citric, succinic acid), sodium phosphate, sodium carbonate or bicarbonate, sodium alginate, sodium bicarbonate, sodium chondroitin sulphate a Na₂HPO₄ aqueous solution and/or a Na₂HPO₄/NaH₂PO₄ aqueous solution.

Water, a Na₂HPO₄/NaH₂PO₄ aqueous solution, a Na₂HPO₄ aqueous solution, a NaCl solution or a sodium citrate solution, are preferred. For example, a solution of 2 to 3% by weight of Na₂HPO₄ in distilled water or a 0.9% NaCl solution can be used.

The pH of the aqueous medium should be between 5 to 10, preferably between 5 and 9, most preferably between 5 and 7.

Preferably, the liquid phase/solid phase (L/S) ratio is between about 0,25 and about 0,7 ml/g, more preferably between about 0,3 and about 0,6 ml/g, the most preferably is about 0,4 ml/g or about 0,5ml/g.

The setting time, which generally ranges from about 10 to about 60 min, preferably about 10 to about 30 min, depends on the composition of the powder and liquid components, the powder-to-liquid ratio, proportion of the calcium phosphate components and the particle sizes of the powder components. The setting time of the cement is an important property of the cement in particular if the cement is intended for use by injection *in situ*. If the setting time is too short, the surgeon does not have time to use the cement before it hardens. If the setting time is too long, the surgeon must wait until he/she can close the wound.

In a preferred embodiment, at least one of the components comprises a setting regulator, a setting accelerator or a setting retarder or both.

A very efficient way to accelerate the setting time is to have large concentrations of phosphate ions in the mixing solution. This can happen via two ways:(i) a soluble phosphate salt is added as a powder in the cement formulation. Upon contact with the mixing solution, the phosphate salt dissolves, and hence accelerates the chemical reaction using up phosphate (LeChatelier principle); (ii) a soluble phosphate salt is pre-dissolved in the mixing liquid phase. Examples of soluble phosphate salts are Na₂HPO₄, NaH₂PO₄, K₂HPO₄, KH₂PO₄, NH₄H₂PO₄. Typical concentrations in the mixing liquid phase are in the range of 0,05 to 1,00 M. Another way to accelerate the setting reaction is to add germs for apatite crystal growth, as the nucleation step of the setting reaction is a limiting factor. Typically, apatite crystals can be used, preferably a calcium-deficient hydroxyapatite or hydroxyapatite powder. Small amounts (a few weight percents) are sufficient to drastically reduce the setting time.

When the setting time is too short, various setting additives can be added to increase the setting time. Typical examples are compounds which inhibit the nucleation and/or growth of apatite crystals. Common examples are pyrophosphate, citrate or magnesium ions. One particularly interesting compound is calcium carbonate. The one skilled in the art may obtain the appropriate setting time with routine assays.

In order to traceany extravasation of the cement into the tissues surrounding bone, it is very important to visualise the cement. The easiest way is to increase the radio-opacity of the cement, for example by means of contrasting agents. For example, metallic powders of tantalum, titanium or tungsten can be used. It might be preferable to use liquid agents in partially bioresorbable cements, such as iodine compounds as iopamidol, iohexol and iotrolan. Preferably, barium sulphate is used.

A further object according to the invention is a drug combined device, in particular a dental or bony implant, or implant coating, comprising a composition according to the invention.

### [Kit]

Another object of the invention is a kit comprising at least a composition according to the invention, an aqueous medium such as a hydrogel (in particular a cellulosic or starch derivated hydrogel) and optionally one or more calcium phosphate compounds.

Injectable calcium phosphate cement compositions can be placed to inaccessible parts of the body and are suited for minimally invasive surgery procedures that reduce damage and pain while hastening return to function. This method of treatment comprises the introduction in the bony defect or fracture through a needle of a suitable calcium phosphate cement.

### [Methods of use]

Another object of the invention is the *in vivo, in vitro* or *ex vivo* use of a composition according to the invention for dental and medical applications relating to bone repair, augmentation, reconstruction, regeneration, and osteoporosis treatment, and also for drug delivery, and as a scaffold for tissue engineering.

The composition according to the invention can also be employed *in vivo, in vitro* or *ex vivo* to produce a dental or a bony implant.

A particularly preferred object of the invention is a dental or a bony implant obtained by moulding of a composition according to the invention.

Main dental applications are: repair of periodontal defects, sinus augmentation, maxillofacial reconstruction, pulp-capping materials, cleft-palate repair, and as adjuvants to dental implants.

Additional medical applications include repair of large bony defects, repair of bone fractures; for spine fusion, surgery revision, bone augmentation, and for bone reconstructions associated with cancer therapy.

In particular, the composition may be useful in orthopaedics, such as in knee surgery: total knee prosthesis ; knee arthroplasty ; osteotomy in particular in connection with iliac auto-graftbone collection., anterior cruciate ligament reconstruction, vertebral fracture reconstruction, foot and ankle surgery, arthrodesis hallux valgus, shoulder surgery such as shoulder replacement.

A particular object of the invention is the use of the composition according to the invention, preferably injectable, for the preparation of a drug-combined device useful for filling a bony defect caused in the iliac crest by collection of auto-graft bone composition.

Iliac crest grafting can be performed on the anterior or posterior iliac crest as need dictates.The anterior crest is used in all cases where the patient is supine. For example, in the treatment of pseudarthrosis of the tibia or in anterior cervical arthrodesis. Graft bone is collected from the anterior iliac crest, at least two finger-widths behind the anterior iliac spine. Cutaneous incision then minimum retraction of the muscles drawn around the bone. The graft is collected using either bone shears or an oscillating saw with a narrow blade. Traditionally, this is a cortico/tri-cortico-cancellous bone graft involving the anterior, posterior and superior cortical bone of the crest. The residual cavity is regular but now only has a floor and the anterior and posterior walls. In some instances of the harvesting of pure cancellous bone, a small hole is made along the upper edge of the crest and the cancellous bone is harvested with a curette. The cavity is therefore impervious on three sides.

The posterior iliac crest is used particularly in spinal surgery when large quantities of bone are required. The patient is in the prone position; the bone is collected from one or both posterior crests, at least two finger-widths outside the sacroiliac articulation. In general, the harvesting involves the "chip" method, using bone shears to remove successive fine shavings of bone. But the method described for the anterior crest may also be employed.

The technique for applying the composition according to the invention in the iliac crest defect caused by bone graft collection depends on the shape of the cavity, its continence and volume: When the defect is small (1 to 5 cm³) and the walls continuous, the composition according to the invention is introduced into the defect, the surface is smoothed with a moist compress and the soft tissue (preferably the periosteum if it can be sutured) is closed up. In case of a large defect (more than 5 cm³) or incontinent cavity: a patch made of an absorbable polymer can be used to reconstruct the shape of the iliac crest. The patch imposes the desired shape while hardening; the cement, in turn, is contained in the newly created impervious cavity. When necessary, screws made of an absorbable polymer may aid in affixing the patch to the bone.

The morbidity for bone collection from the iliac crest has been widely described. The principal disadvantages are post-operative pain, which is often intense, hematomas at the harvesting site and, in the longer term, cosmetic consequences or even hernias of the abdominal viscera through a more or less sizeable harvesting. Nevertheless, the (anterior or posterior) iliac crest constitutes a genuine autogenous bone bank, the osteoconductive and osteogenetic qualities of which remain difficult to match using other types of grafts or substitutes. The filling of the bony defect caused by auto-graft collection in the iliac crest with a composition according to the invention limits the morbidity of the grafting and allows local hemostasis, analgesia and bone reconstruction of the defect created by the collection.

Iliac crest grafting is always accompanied by bone bleeding, which may be the cause of a painful post-operative hematoma that, in some cases, requires re-intervention for the purposes of drainage. The composition according to the invention applied to the collection graft site ensures local hemostasis linked to the cohesive characteristics of the cement.

Pain at the collection graft site is treated through the release of the analgesic contained in the composition according to the invention. The release is effective up to 96 hours after the intervention: i.e., during the most painful phase of the graft harvesting.

The application of the composition according to the invention helps also to achieve two important goals: immediate reconstruction of the loss of bone substance and replenishment, in the middle term, of bone capital. Immediate reconstruction helps prevent a certain number of painful, local complications over loss of bone substance (difficulty wearing a belt) and fragilization of the crest with a risk of fracture (capable, in extreme cases, of radiating out towards the sacroiliac articulation in back, the anterior superior iliac spine in front or the roof of the acetabulum below). Replenishment of bone capital is a fundamental point since composition according to the invention, as it is absorbed, helps to recapitalize available bone stock, permitting other graft collections, when necessary, from the same site in the middle term.

The following drawings and examples are given to illustrate and describe specific aspects and preferred embodiments of the invention.
- **Fig.1:**: Bupivacaine calibration range at 270 nm
- **Fig.2:**: Bupivacaine and lidocaine release kinetics from CDA
- **Fig.3:**: Post-operative recovery: Von Frey monofilament
- **Fig.4:**: Post-operative recovery: neurological score
- **Fig.5:**: Post-operative recovery: inflammatory process

### EXAMPLES

### EXAMPLE 1: CDA-bupivacaine and CDA-lidocaine association

One dose of lidocaine was assayed : 5% w/w.

Three doses of bupivacaine were assayed : 1%, 4% and 16% w/w, i.e. 0,25mg, 1mg and 4mg of bupivacaine for an implant of 25mg.

The active principle bupivacaine was first diluted in ethanol and the appropriate amount of active principle is added to the CDA powder (synthesized according to reference 12, particle size 40-80 µm). The mixture was then mixed at room temperature during one hour at a speed of 50 rpm using a Rotator drive STR4 from Stuart Scientific. After mixing, the ethanol wzs removed by lyophilisation using appropriate equipment (Christ alpha1-4 from Bioblock Scientific).

The powder thus obtained wzs compressed on a cold isostatic press (FF558 from NovaSwiss) by isostatic compression of 140 MPa during 5 minutes. This product is called "CDA-bupivacaine without compression".

Part of the blocks obtained were subsequently crushed in a mortar made of alumina to an approximate mean particle size of 200 nm. The products obtained are called in the following "CDA-bupivacaine" and "CDA-lidocaine", respectively.

### EXAMPLE 2: Analgesic release kinetics

### Assay methods:

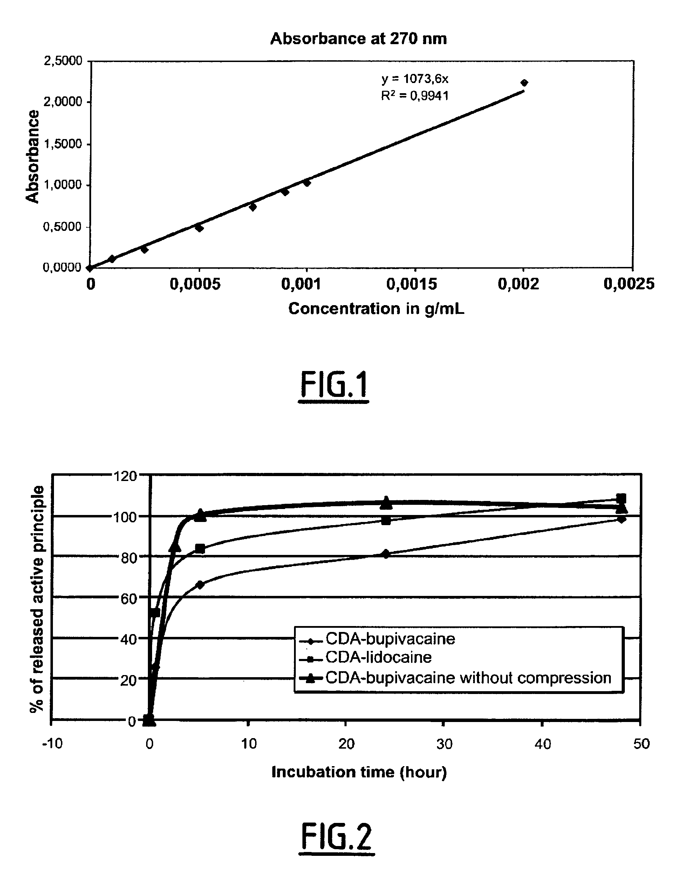

First, a method for assaying the bupivacaine release was developed. The released bupivacaine is assayed by UV spectrophotometry. Several wavelengths were tested. At short wavelengths (200 nm), the assay is more sensible (about 1 µg/mL) but the result may be affected by the presence of phosphate ions released by the CDA. On the contrary, at long wavelengths (262-270nm), the phosphate ions absorbance does not interfere with the bupivacaine absorbance. Consequently, bupivacaine was thus assayed at 270nm (see Fig. 1).

The same method was applied to determine the lidocaine release. A first assay confirmed that the bupivacaine within the composition is stable for 3 months at 4°C.

### Release kinetics:

200 mg of CDA powder as prepared in Example 1 were introduced in distilled water (15mL) at 37°C while mixing. After an incubation time of 30 min, 2h30, 5h, 24h, 48h, 5 days, 2mL liquid were removed, filtered and assayed by UV spectrophotometry. The removed liquid were replaced by 2 mL of distilled water.

The results are shown in Fig. 2. They indicate that CDA-bupivacaine and CDA-lidocaine have similar release kinetics. However, lidocaine is released faster than bupivacaine. 53% of lidocaine are released in the first 30 min vs. 26% of bupivacaine. Both lidocaine and bupivacaine are totally released in 48 hours. 85,6% in weight of bupivacaine are released from "CDA-bupivacaine without compression" in 2h30.

### EXAMPLE 3: Post-operative analgesic effect of CDA-bupivacain knee implant in rat

### Animals

50 Wistar male rats weighing between 250 and 275 g on their arrival to the animal facilities were used. After handling in order to get accustomed to the investigator presence, animals were placed by group of two into polycarbonate transparent F1 type cages with dust free wood shavings bedding (Safe) and free access to water and food, in the animal room with controlled temperature (21°C±1°C), hygrometry (45%±10%) and light/dark cycle (light 7h to 19h).

After a 5 days adjustment period, surgery was performed. Each animal was tagged with an identification number on the tail. Surgery was performed at the animal facilities operating bloc of the medical school of Nîmes, France.

### Reference substance for the model

The CDA-bupivacaine powder obtained in Example 1 is used to fill a cylindrical rat knee defect with 3mm in diameter and 5mm in length. The powder was dropped directly inside the defect using a sterile cone.

### Analgesia measurement

Von Frey monofilament (electronic version) can be used to determine analgesia threshold of the awaken animal when the arch of the foot is submitted to an increasing mechanical pressure. The pressure is provided by the investigator and leg retraction threshold, expressed in grams, corresponds to the analgesia threshold. The mediolateral distance of the implanted knee, measured with a sliding calliper, can be used to obtain quantitative information directly related to the inflammation and tumefaction degree of the operated area.

Different qualitative observations were scored by the investigator in order to obtain an estimation of the animal analgesia state:
- joints movement;
- holding on the leg;
- leg position (dorsal vs. plantar);
- leg position (rotation vs. parallel); and
- wound aspect at the implantation site.

Each parameter will be scored from 0 to 2 with:
- 0: maximal discomfort of the animal
- 1: partial discomfort of the animal
- 2: no discomfort

The total score is thus included between 0 (major handicap) and 12 (normal locomotion).

### Experimental procedure

Five groups of 10 rats each were included in the study:
- Group 1: control lot (surgery control)
- Group 2: positive control lot ("naked" implant)
- Group 3: 1 mg Bupivacaine implant lot
- Group 4: 4 mg Bupivacaine implant lot
- Group 5: 16 mg Bupivacaine implant lot

### Procedure

Von Frey monofilament: Bilateral quantification of mechanical hyperalgesia was performed four times on the surgery day J0 (30; 60; 120 and 240 min after the animal recovery) then once a day during the 3 days following operation J1, J2 and J3.

Mediolateral distance of the implanted knee: Mediolateral distance measurement of the operated knee was performed a first time on the surgery day J0 (at 240 min following operation) then once a day during the 3 days following operation J1, J2 and J3.

Neurological score: A neurological score was attributed to each animal (non-operated and operated leg) concurrently with Von Frey monofilament test, i. e. 30, 60, 120 and 240 min after the animal recovery on the surgery day J0 then once a day during the 3 days following operation J 1, J2 and J3..

### Results

Animal performances calculation were performed and expressed as follows for each of the experimental groups:

Von Frey monofilament: Analgesia threshold expressed in grams (mean± S.E.M) (cut-off fixed at 150g). Percentage of operated leg recovery relative to the mean value for the non-operated leg (percentage±S.E.M)

Mediolateral distance of the implanted knee: Mediolateral distance of knee expressed in mm (mean± S.E.M). Percentage of calculated distance variation relative to the mean value of the non-operated knee distance (percentage±S.E.M)

Neurological score: Score expressed in score unit (mean± S.E.M). Percentage of calculated score variation relative to the mean value of the non-operated leg score (percentage±S.E.M)

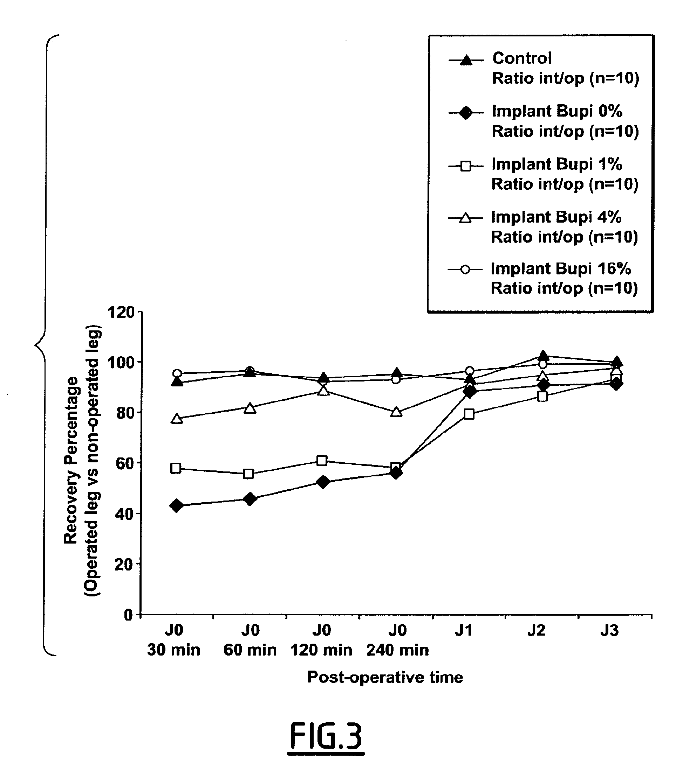

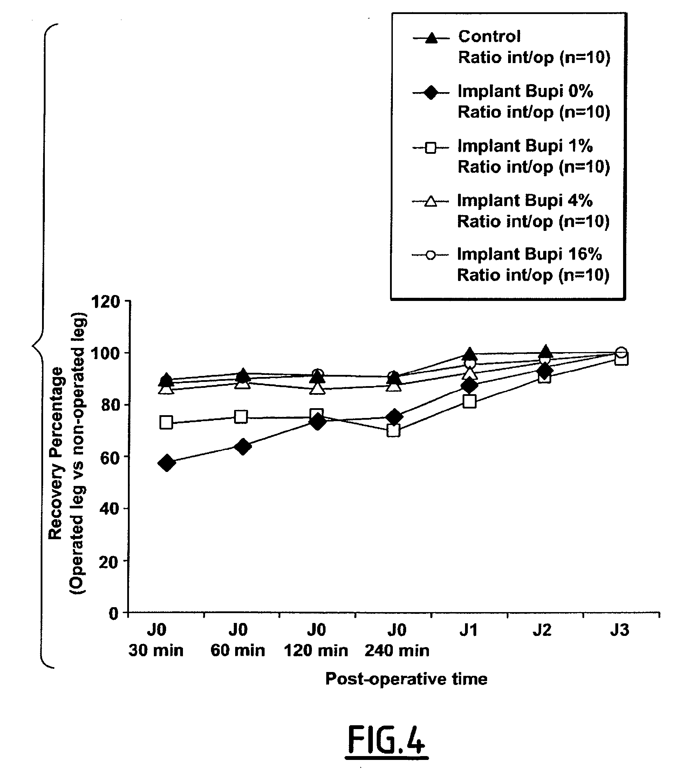

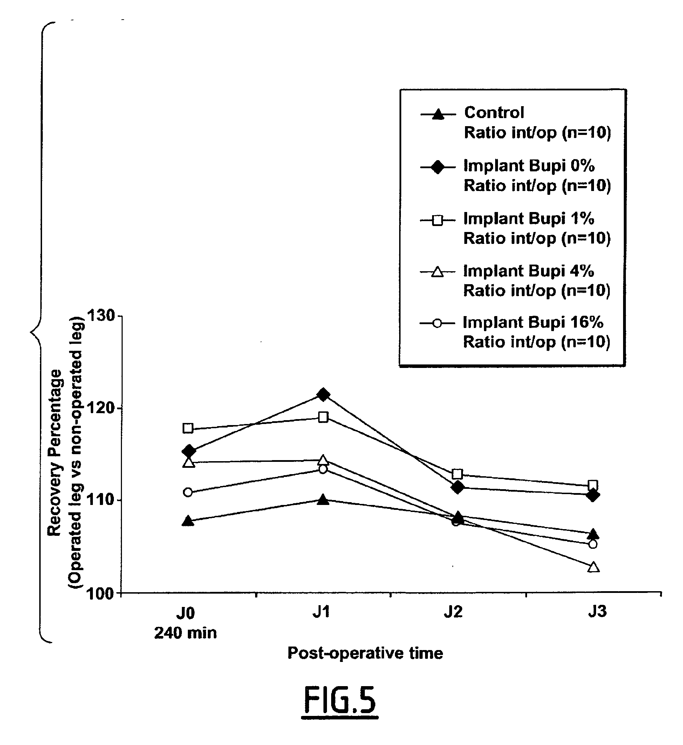

Once the data was averaged for all the animals of the same experimental group, the significance of the observed effects (between different groups or between different treatments) was tested using variance analysis (ANOVA) through a statistical program. The analysis involves the comparison of the different animal groups. Where global ANOVA is significant, Dunnett post-hoc test is used for the adequate inter-groups comparisons. The threshold for significance was set at 95% (p<0.05) or at 99% (p<0.01) (see Fig. 3 to 5 and the following Table I).

**Table I: Analgesia measurements on rat**

| Measures | Inflammatory process | Neurological score | Von Frey |
|---|---|---|---|
| 0% vs 1% | NS | NS | ***(10⁻⁴) |
| 0% vs 4% | **(10⁻³) | ***(10⁻¹⁴) | ***(10⁻⁸) |
| 0% vs 16% | **(10⁻³) | ***(10⁻¹⁶) | **(10⁻³) |
| 1 % vs 4% | ***(10⁻⁴) | ***(10⁻⁸) | ***(10⁻⁶) |
| 4% vs 16% | NS | NS | NS |

| | | | |
|---|---|---|---|
| **** p<0.05** ***** p<0.01** | | | |

### Post-mortem analysis

Operated femurs were collected for study and sent for histological analysis to the veterinary school of Nantes. In rat, bupivacaine adsorbed on resorbable implant, induces a significatively dose-dependant antalgic effect when compared with animals having received bupivacaine-free implant. This effect is transitory since it disappears at J+1 following operation and is stronger with regard to mechanic hypersensitivity.

### REFERENCES

1. Butterworth JF, Strichartz GR. Molecular mechanisms of local anesthesia: a review. Anesthesiology 1990;72:711-34
2. Li Y-M, Wingrove DE, Too HP, Marnerakis M, Stimson ER, Strichartz GR et al. Local anesthetics inhibit substance P binding and evoked increases in intracellular Ca2+. Anesthesiology 1995;82:166-73
3. Kaba A, Laurent SR, Detroz BJ, Sessler DI, Durieux ME, Lamy ML, Joris JL : Intravenous lidocaine infusion facilitates acute rehabilitation after laparoscopic colectomy. Anesthesiology. 2007 Jan;106(1):11-8; discussion 5-6.
4. C. Chenu. Innervation de l'os. Médecine/sciences 2001 ; 17 : 1276-80
5. M Gentili. in Anesthésie pour chirurgie orthopédique JEPU 1997
6. Bigby J, Reader A, Nusstein J, Beck M, Weaver J. Articaine for supplemental intraosseous anesthesia in patients with irreversible pulpitis. J Endod. 2006 Nov;32(11):1044-7. Epub 2006 Jul 26.
7. Gallatin J, Reader A, Nusstein J, Beck M, Weaver J. A comparison of two intraosseous anesthetic techniques in mandibular posterior teeth. J Am Dent Assoc. 2003 Nov;134(11):1476-84.
8. Sesay M, Dousset V, Liguoro D, Péhourcq F, Caillé JM, Maurette P. Intraosseous lidocaine provides effective analgesia for percutaneous vertebroplasty of osteoporotic fractures. Can J Anaesth. 2002 Feb;49(2):137-43
9. Nechleba J, Rogers V, Cortina G, Cooney T. Continuous intra-articular infusion of bupivacaine for postoperative pain following total knee arthroplasty. J Knee Surg. 2005 Jul; 18(3): 197-202.
10. Hoeft MA, Rathmell JP, Dayton MR, Lee P, Howe JG, Incavo SJ, Lawlis JF. Continuous, intra-articular infusion of bupivacaine after total-knee arthroplasty may lead to potentially toxic.
11. Le Thiesse J.C., Caractéristiques morphologiques et surfaciques des matières premières pulvérulentes. Evaluation et application, STP Pharm., 1990, 6(3):169-80
12. L. Obadia, T. Rouillon, B. Bujoli, G. Daculsi, J.-M. Bouler "Calcium Deficient Apatites synthesized by ammonia hydrolysis of dicalcium phosphate dihydrate: influence of temperature, time and pressure" J. Biomed. Mater.Res. B. (2006) 80B(1):32-42.

## Claims

1. A composition useful as bone substitute comprising one or more calcium-phosphate compounds in association with an analgesic.

2. The composition according to claim 1, wherein the composition is in form of a powder.

3. The composition according to claim 1 or 2, wherein the composition has a specific BET area between about 1 000 m².kg⁻¹ and about 300 000 m².kg⁻¹.

4. The composition according to any of claims 1 to 3, wherein at least one calcium-phosphate compound is selected from the group consisting of CDA, DCPA, DCPD, TTCP or a mixture thereof.

5. The composition according to any of claims 1 to 4, further comprising a sintered calcium-phosphate compound selected from the group consisting of HA, α-TCP, β-TCP, and BCP.

6. The composition according to any of claims 1 to 5, comprising at least 80% by weight of α-TCP.

7. The composition according to any of claims 1 to 6, wherein the analgesic is a morphine related substance.

8. The composition according to any of claims 1 to 6, wherein the analgesic is a local anaesthetic.

9. The composition according to claim 8, wherein the local anaesthetic is an aminoamide or an aminoester.

10. The composition according to claim 9, wherein the amino ester is chosen from benzocaine, chloroprocaine, cocaine, procaine and tetracaine.

11. The composition according to claim 9, wherein the aminoamide is chosen from bupivacaine, levobupivacaine, lidocaine, mepivacaine, prilocaine, ropivacaine, articaine and trimecaine.

12. A preparation process of a composition according to any of claims 1 to 11, comprising the following steps:
a) providing a calcium-phosphate powder, a liquid and an analgesic ;
b) mixing of the components to obtain a suspension; and
c) removing the liquid from the suspension to obtain a solid; and
d) optionally compressing and grinding the solid obtained into a calcium-phosphate powder charged with the analgesic.

13. The process according to claim 12, wherein step (c) is conducted by lyophilisation.

14. The process according to claim 12 or 13, wherein step (d) comprises the compression of the solid at a pressure of between about 50 MPa to about 500 MPa.

15. The process according to any of claims 12 to 14, wherein the liquid is non aqueous.

16. The process according to any of claims 12 to 15, wherein the calcium-phosphate powder in step (a) has a mean particle size comprised between about 0,2 µm and about 100 µm.

17. The process according to any of claims 12 to 16, wherein the suspension in step (b) comprises from about 0,5 to about 20 % by weight of analgesic.

18. The process according to any of claims 12 to 17, wherein the calcium-phosphate powder charged with the analgesic obtained in step (d) has a mean particle size between about 1 µm and about 500 µm.

19. A composition obtainable according to the process of any of claims 12 to 18.

20. A preparation process of a drug-combined device comprising the following steps:
(i) mixing of a composition according to any of claims 1 to 11 and 19 with an appropriate amount of an aqueous medium ;
(ii) putting the mixture into a suitable form ; and
(iii) setting of the mixture into a solid drug-combined device.

21. The process according to claim 20, wherein the mixture in step (i) is in a form suitable to be injected.

22. Drug combined device comprising a composition according to any of claims 1 to 11 and 19.

23. A drug combined device according to claims 1 to 22, wherein the drug combined device is a dental or bony implant or implant coating.

24. Kit comprising a composition according to any of claims 1 to 11 and 19, an aqueous medium and optionally further one or more calcium phosphate compounds.

25. A kit according to claim 24, wherein the aqueous medium is a hydrogel.

26. Use of a composition according to any of claims 1 to 11 and 19 for the preparation of a drug-combined device useful for filling a bony defect caused in the iliac crest by collection of auto-graft bone.

27. Use *in vitro* or *ex vivo* of a composition according to any of claims 1 to 11 and 19 as a scaffold for tissue engineering.

28. Use *in vitro* or *ex vivo* of a composition according to any of claims 1 to 11 and 19 to produce a dental or bony implant.
